# EUROPÄISCHE PATENTSCHRIFT

(11) **EP 3 986 517 B1**
(45) Veröffentlichungstag und Bekanntmachung des Hinweises auf die Patenterteilung: **01.10.2025**
(21) Anmeldenummer: 20728669.1
(22) Anmeldetag: 18.05.2020
(51) Int. Cl.: A61M 16/00, F04D 25/08, F04D 29/42

(54) **LÜFTEREINHEIT FÜR EIN BEATMUNGSGERÄT**
VENTILATION UNIT FOR A VENTILATOR
UNITÉ DE VENTILATION POUR UN VENTILATEUR

(30) Priorität: 20.06.2019 DE 102019116718
(43) Veröffentlichungstag der Anmeldung: 27.04.2022
(73) Patentinhaber: Löwenstein Medical Technology S.A., 2557 Luxembourg (LU)
(72) Erfinder: GERLACH, Angela, 22607 Hamburg (DE)
(74) Vertreter: Löwenstein Medical IP
(86) Internationale Anmeldenummer: PCT/EP2020/025230
(87) Internationale Veröffentlichungsnummer: WO 2020/253983

(56) Entgegenhaltungen:
- EP-A1- 2 947 328
- WO-A1-2014/178475
- WO-A1-2019/014173
- US-A1- 2017 130 723
- US-A1- 2018 064 894
- US-B2- 10 323 650
- US-B2- 8 973 576

## Beschreibung

Die vorliegende Erfindung betrifft eine Lüftereinheit für ein Beatmungsgerät, insbesondere eine Lüftereinheit mit einem Gehäuse und einem in dem Gehäuse drehbar gelagerten Lüfterrad, mit welchem durch Rotation ein Fluid durch einen Einlasskanal des Gehäuses ansaugbar und anschließend durch einen Auslasskanal des Gehäuses wieder ausstoßbar ist.

Derartige Lüftereinheiten für Beatmungsgeräte zur Beatmung insbesondere von Personen mit unzureichender oder ausgesetzter Eigenatmung sind in unterschiedlichen Ausführungen bekannt. Beispielsweise beschreibt die EP 2 947 328 A1 eine Lüftereinheit mit einem Elektromotor, einem von diesem angetriebenen Flügelrad sowie einem das Flügelrad und den Elektromotor aufnehmenden Gehäuse. Die vorbekannte Lüftereinheit ist eine freiansaugende Lüftereinheit, das heißt sie weist saugseitig keinen Saugstutzen auf, so dass das Atemgas nicht über einen saugseitig mit der Lüftereinheit verbundenen Schlauch angesaugt wird. Diese freiansaugende Lüftereinheit kann beispielsweise in einer Gebläsebox des Beatmungsgeräts aufgenommen sein. US 2017/0130723 A1 offenbart eine als Trommelläufer ausgebildete Lüftereinheit für Klimaanlagen.

Vor diesem Hintergrund liegt der vorliegenden Erfindung die Aufgabe zugrunde, eine Lüftereinheit für ein Beatmungsgerät bereitzustellen, die eine verbesserte Betriebsleistung aufweist und bezieht sich auf eine freiansaugende Lüftereinheit und auch auf eine nichtfreiansaugende Lüftereinheit. So soll insbesondere ein saugseitig mittels des Lüfterrads in das Gehäuse gesaugtes Fluid, zum Beispiel ein Atemgas, druckseitig mit einem vorherbestimmten Druck wieder aus dem Gehäuse ausströmbar sein, wobei der Ausströmdruck der Lüftereinheit im Vergleich zum Ausströmdruck herkömmlicher Lüftereinheiten für Beatmungsgeräte bei derselben Drehzahl des Lüfterrads höhere Werte erreichen soll bzw. die Drehzahl der Lüftereinheit bei gleichem Ausströmdruck der Lüftereinheit im Vergleich zu herkömmlichen Lüftereinheiten reduzierbar sein soll.

Diese Aufgabe wird durch eine Lüftereinheit mit den Merkmalen des Anspruchs 1 gelöst. Weitere, besonders vorteilhafte Ausgestaltungen der Erfindung offenbaren die Unteransprüche.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Die Erfindung ist durch die beigefügten Ansprüche definiert.

Erfindungsgemäß weist eine Lüftereinheit für Beatmungsgeräte zur Beatmung beispielsweise von Personen mit unzureichender oder ausgesetzter Eigenatmung ein Gehäuse mit einem Saugstutzen auf, wobei der Saugstutzen einen Einlasskanal zum saugseitigen Einströmen eines Fluids, zum Beispiel eines Atemgases, in das Gehäuse bildet. Das Gehäuse weist weiter einen Druckstutzen auf, der einen Auslasskanal zum druckseitigen Ausströmen des Fluids aus dem Gehäuse bildet. Ferner weist die Lüftereinheit gemäß der Erfindung ein in dem Gehäuse drehbar gelagertes Lüfterrad auf. Dieses ist ausgebildet und angeordnet, durch Rotation das Fluid über den Saugstutzen anzusaugen und durch den Einlasskanal in das Gehäuse zu fördern sowie anschließend das Fluid über den Druckstutzen wieder auszustoßen und durch den Auslasskanal aus dem Gehäuse zu fördern. Es ist zu verstehen, dass die Rotation des Lüfterrads beispielsweise motorisch, vorzugsweise mittels eines Elektromotors, bewirkt sein kann, ohne jedoch hierauf beschränkt zu sein. Eine manuell angetriebene Rotation des Lüfterrads ist gleichfalls denkbar.

Der Saugstutzen weist eine dem Einlasskanal zugewandte Innenfläche auf. Mit anderen Worten begrenzt die Innenfläche des Saugstutzens den Einlasskanal in radialer Richtung nach außen. Somit ist durch die Innenfläche des Saugstutzens ein Einlasskanalnenndurchmesser bestimmt. Erfindungsgemäß weist der Saugstutzen an seiner Innenfläche wenigstens ein Strömungsleitelement auf, welches einen vom Einlasskanalnenndurchmesser abweichenden Einlasskanalinnendurchmesser festgelegt. Als abweichend im Sinne der vorliegenden Erfindung ist hierbei ein solcher Größenunterschied zwischen den beiden Kanaldurchmessern zu verstehen, dass dieser sicher nicht in den fertigungsbedingten Toleranzbereich eines Saugstutzens fällt, sondern vielmehr das Ergebnis zielgerichteten Handelns ist. Herstellungsbedingte Größenabweichungen der beiden vorgenannten Kanaldurchmesser, die innerhalb der für die Fertigung des Saugstutzens festgelegten, herkömmlichen Fertigungstoleranzen liegen, sind folglich nicht geeignet, einen Größenunterschied zwischen dem Einlasskanalnenndurchmesser und dem Einlasskanalinnendurchmesser im Sinne der Erfindung zu begründen.

Im Gegensatz zu einer glatten, ebenen Innenfläche eines herkömmlichen Saugstutzens weist der Saugstutzen der Lüftereinheit gemäß der Erfindung folglich keine ebene Innenfläche auf. Vielmehr wird die Innenfläche des erfindungsgemäßen Saugstutzens durch das wenigstens eine Strömungsleitelement strukturiert, wodurch die Strömung des angesaugten Fluids, zum Beispiel ein Gas bzw. Atemgas, in der Kontur des Strömungsleitelements entsprechenden Weise beeinflussbar, das heißt steuerbar ist. Die nicht mit dem Strömungsleitelement versehenen, den Einlasskanalnenndurchmesser bestimmenden Bereiche der Innenfläche des Saugstutzens können weiterhin glatt und eben ausgebildet sein, ohne jedoch zwingend hierauf beschränkt zu sein.

Insbesondere ist allein durch das Vorsehen des wenigstens einen Strömungsleitelements zur lokalen Veränderung des Einlasskanalnenndurchmessers eine Druckerhöhung des durch das Lüfterrad angesaugten Fluids erzielbar. Dies wiederum ermöglicht bei im Vergleich zu herkömmlichen Lüftereinheiten gleichbleibender Drehzahl des Lüfterrads der erfindungsgemäßen Lüftereinheit eine Erhöhung des Drucks des druckseitig ausströmenden Fluids. Alternativ kann bei im Vergleich zu herkömmlichen Lüftereinheiten gleichbleibendem Fluiddruck im Auslasskanal des Druckstutzens die Drehzahl des Lüfterrads bei der erfindungsgemäßen Lüftereinheit auch verringert werden. Hierdurch kann die Betriebseffizienz der Lüftereinheit gemäß der Erfindung verbessert werden, durch eine Verringerung der Drehzahl können die Laufruhe des Lüfterrads erhöht werden, Vibrationen, mechanischer Verschleiß z. B. an Lüfterradlagern und eine Geräuschentwicklung der Lüftereinheit verringert werden.

Die Lüftereinheit gemäß der Erfindung ist durch den saugseitig vorgesehenen Saugstutzen, der vom Gehäuse als solches durch eine den Einlasskanal bildende längliche Erstreckung abgegrenzt ist und zum Beispiel aus der eigentlichen Außenkontur des Gehäuses hervorstehen kann bzw. aus diesem herausragt, keine freiansaugende Lüftereinheit. Vielmehr kann der Saugstutzen mit einem Schlauch verbunden sein, über den das Fluid in den Einlasskanal angesaugt wird.

Das Gehäuse der vorliegend offenbarten Lüftereinheit kann eine im Wesentlichen geschlossene Gehäuseeinheit sein, die das Lüfterrad derart umgibt, dass die Fluidströmung wie hierin beschrieben, nämlich Ansaugen des Fluids auf der Saugseite der Lüftereinheit und Ausstoßen des Fluids auf der Druckseite der Lüftereinheit, insbesondere mit einem gegenüber der Saugseite erhöhten Fluiddruck, durch die Rotation des Lüfterrads erzeugt werden kann. In dem Gehäuse kann zusätzlich auch ein das Lüfterrad antreibender Motor, sofern vorgesehen, zum Beispiel ein elektromotorisch betriebener Antriebsmotor, aufgenommen sein. Dies ist jedoch nicht zwingend erforderlich.

Die Querschnittsfläche des Einlasskanals kann eine runde Form, z. B. kreisförmig, elliptisch und dergleichen, aufweisen. Sie kann auch eine eckige Form, zum Beispiel rechteckig, quadratisch, trapezförmig, allgemein polygonal und dergleichen haben.

Gemäß einer vorteilhaften Ausgestaltung der Erfindung ist das Strömungsleitelement eine von der Innenfläche des Saugstutzens hervorstehende Rippe (hierin auch als Finne, Steg, Lamelle und dergleichen bezeichnet). Mit anderen Worten ragt die Rippe von der Innenfläche in radialer Richtung nach innen in den Einlasskanal hinein. Dementsprechend verkleinert die Rippe den durch sie lokal festgelegten Einlasskanalinnendurchmesser gegenüber dem von den nicht mit der Rippe versehenen Bereichen der Innenfläche festgelegten Einlasskanalnenndurchmesser. Der Einlasskanalinnendurchmesser ist hierbei durch den Abstand zwischen einer in radialer Richtung am weitesten innen in den Einlasskanal ragenden Stirnseite der Rippe und der den Einlasskanal diametral gegenüberliegend begrenzenden Begrenzungsfläche festgelegt. Diese Begrenzungsfläche kann die den Einlasskanalnenndurchmesser bestimmende Innenfläche des Saugstutzens sein, sie kann jedoch ebenfalls eine Fläche eines weiteren Strömungsleitelements sein, das der erstgenannten Rippe diametral gegenübersteht.

Gemäß einer weiteren vorteilhaften Ausgestaltung der Erfindung ist das Strömungsleitelement eine in die Innenfläche des Saugstutzens eingebrachte Nut. Die Nut, die eine in radialer Richtung nach außen in die Innenfläche eingebrachte Vertiefung darstellt, vergrößert dementsprechend den Einlasskanalinnendurchmesser lokal gegenüber dem von den nicht mit der Nut versehenen Bereichen der Innenfläche festgelegten Einlasskanalnenndurchmesser. Der Einlasskanalinnendurchmesser ist hier durch den Abstand in radialer Richtung zwischen einem in radialer Richtung weiter außen liegenden Nutgrund und einer den Einlasskanal diametral gegenüberliegend begrenzenden Begrenzungsfläche festgelegt. Diese Begrenzungsfläche kann die den Einlasskanalnenndurchmesser bestimmende Innenfläche des Saugstutzens sein, sie kann jedoch gleichfalls eine Fläche eines weiteren Strömungsleitelements sein, das der erstgenannten Nut diametral gegenübersteht.

Das als Nut oder Rippe ausgebildete Strömungsleitelement ermöglicht aufgrund der lokalen Verkleinerung bzw. Vergrößerung des Kanalinnendurchmessers gegenüber dem Einlasskanalnenndurchmesser eine unterschiedliche, gezielte Beeinflussung der Fluidströmung, insbesondere Druckerhöhung, im Einlasskanal. Es hat sich herausgestellt, dass ein als Nut ausgebildetes Strömungsleitelement die Druckkennlinie der Fluidströmung stärker beeinflusst als ein als Rippe ausgebildetes Strömungsleitelement.

Nach einer weiteren vorteilhaften Ausgestaltung sind mehrere Strömungsleitelemente äquidistant, d. h. in gleichen Abständen zueinander, über den Umfang der Innenfläche des Saugstutzens verteilt angeordnet. Durch die Anzahl der insgesamt an der Innenfläche umfänglich verteilt angeordneten Strömungsleitelemente lässt sich die Fluidströmung und damit der Fluiddruck, insbesondere eine Druckerhöhung, im Einlasskanal weiter gezielt beeinflussen.

Eine noch weitere vorteilhafte Ausgestaltung der Erfindung sieht vor, dass sich bei Vorsehen mehrerer an der Innenfläche des Saugstutzens umfänglich verteilt angeordneten Rippen als Strömungsleitelemente diese Rippen in radialer Richtung bis zur Mittelachse (hierin auch als Symmetrieachse bezeichnet) des Saugstutzens erstrecken und dort miteinander in Kontakt stehen. Sie können an ihrem Kontaktpunkt im Einlasskanal stofflich miteinander verbunden sein, was jedoch nicht zwingend erforderlich ist. Mit anderen Worten bilden die Rippen im Querschnitt des Saugstutzens gesehen wenigstens einen den Einlasskanal in radialer Richtung vollständig querenden Steg (bei zwei diametral angeordneten und sich berührenden Rippen) oder eine sternförmige Anordnung (bei drei, vier, fünf oder mehr verteilt angeordneten Rippen). Es ist darauf hinzuweisen, dass bei der vorliegenden Ausgestaltung mit sich auf der Mittelachse berührenden Rippen der durch diese bestimmte, im Vergleich zum Einlasskanalnenndurchmesser verkleinerte Einlasskanalinnendurchmesser seinen absolut minimalen Wert annimmt, nämlich den Wert Null.

Gemäß einer noch weiteren vorteilhaften Ausgestaltung der Erfindung erstreckt sich das Strömungsleitelement in seiner Längserstreckungsrichtung parallel zur Mittelachse des Saugstutzens. Mit anderen Worten ist das Strömungsleitelement in seiner Längserstreckungsrichtung achsparallel zur Mittelachse des Saugstutzens ausgerichtet. Hierdurch lässt sich die durch das Strömungsleitelement bewirkte Druckänderung, insbesondere Druckerhöhung, des durch den Saugstutzen strömenden Fluids besonders effektiv erzielen.

Gemäß einer anderen vorteilhaften Ausgestaltung der Erfindung erstreckt sich das Strömungsleitelement in seiner Längserstreckungsrichtung in einem Winkel geneigt zur Mittelachse des Saugstutzens, dementsprechend also nicht achsparallel zur Mittelachse. Je nach Neigungswinkel der Längserstreckung des Strömungsleitelements kann die durch diese bewirkte Druckerhöhung im Einlasskanal sehr präzise gesteuert werden, insbesondere gegenüber der achsparallelen Anordnung in gewünschter Weise gezielt abgeschwächt werden.

Nach einer weiterhin bevorzugten Ausgestaltung der Erfindung ist der vorerwähnte Winkel bzw. Neigungswinkel der Längserstreckung des Strömungsleitelements gegenüber der Mittelachse des Saugstutzens bzw. Einlasskanals größer 0 ° und kleiner gleich 45 °. Es ist zu verstehen, dass die Längserstreckungsrichtung des Strömungsleitelements bei dieser Ausgestaltung sich im Sinne der Erfindung immer noch hauptsächlich in Richtung der Mittelachse des Saugstutzens erstreckt. Weiterhin ist zu verstehen, dass je nach Definition des Neigungswinkels gegenüber der Mittelachse des Saugstutzens der Winkelbereich kleiner 0 ° bis größer gleich -45 ° als äquivalent zum vorstehend angegebenen Winkelbereich größer 0 ° und kleiner gleich 45 ° anzusehen ist.

Nach einer weiterhin vorteilhaften Ausgestaltung der Erfindung liegt das Verhältnis des Einlasskanalinnendurchmessers zum Einlasskanalnenndurchmesser zwischen 0,6 und 1,4, bevorzugt zwischen 0,7 und 1,3, noch bevorzugter zwischen 0,75 und 1,25. Es hat sich herausgestellt, dass sich mit diesen Verhältnissen die gewünschten Druckänderungen im Einlasskanal des Saugstutzens am effektivsten erzielen lässt. Es ist zu verstehen, dass ein Verhältnis des Einlasskanalinnendurchmessers zum Einlasskanalnenndurchmesser kleiner Eins durch das Vorsehen von wenigstens einem rippen-/stegförmigen Strömungsleitelement zu erreichen ist, ein Verhältnis des Einlasskanalinnendurchmessers zum Einlasskanalnenndurchmesser größer Eins durch das Vorsehen von wenigstens einem nutförmigen Strömungsleitelement.

Weiterhin bevorzugt ist die Lüftereinheit nach einer Ausgestaltung eine Radiallüftereinheit, das heißt das Fluid wird von dem Lüfterrad in seiner Axialrichtung angesaugt und in seiner Radialrichtung ausgestoßen.

In manchen Ausgestaltungen der Erfindung weist das Lüfterrad der Lüftereinheit eine Mehrzahl an Schaufelelementen auf, wobei die Schaufelelemente wenigstens teilweise mit jeweils einem an wenigstens einer axialen Längsseite des Schaufelelements wenigstens abschnittsweise verlaufenden Winglet ausgestattet sind.

In manchen Ausgestaltungen der Erfindung weist das Winglet dabei eine Ausdehnung von 1° bis 20° des Umfangs des Lüfterrades auf.

Die Ausdehnung des Winglets nimmt in manchen Ausgestaltungen der Erfindung von radial innen des Lüfterrades nach radial außen des Lüfterrades zu.

In manchen Ausgestaltungen der Erfindung ist das Lüfterrad an nur einer axialen Seite mit wenigstens einer Scheibe ausgestattet und die Scheibe ist nur an derjenigen axialen Seite angeordnet, welche einer mit den Winglets ausgestatteten axialen Seite des Lüfterrades gegenüberliegt.

Weitere Merkmale und Vorteile der Erfindung ergeben sich aus der folgenden Beschreibung nicht einschränkend zu verstehender Ausführungsbeispiele der Erfindung, die im Folgenden unter Bezugnahme auf die Zeichnung näher erläutert wird. In dieser Zeichnung zeigen schematisch:
- Fig. 1: eine Explosionsansicht eines Ausführungsbeispiels einer Lüftereinheit gemäß der Erfindung,
- Fig. 2: eine perspektivische Teilansicht der Lüftereinheit aus Fig. 1,
- Fig. 3: eine perspektivische Teilansicht eines weiteren Ausführungsbeispiels einer Lüftereinheit gemäß der Erfindung,
- Fig. 4(a)-(c): eine perspektivische Detailansicht eines Saugstutzens der Lüftereinheit aus Fig. 2, eine perspektivische Detailansicht eines Saugstutzens eines weiteren Ausführungsbeispiels einer Lüftereinheit gemäß der Erfindung sowie eine perspektivische Detailansicht eines Saugstutzens eines noch weiteren Ausführungsbeispiels einer Lüftereinheit gemäß der Erfindung,
- Fig. 5: eine Querschnittansicht durch den Saugstutzen der Lüftereinheit aus Fig. 2 und
- Fig. 6: ein Druckdiagramm, das einen Vergleich der Lüftereinheit aus Fig. 2 mit einer Lüftereinheit nach dem Stand der Technik darstellt.
- Fig. 7: eine perspektivische Ansicht eines Ausführungsbeispiels einer Lüftereinheit.
- Fig. 8: eine perspektivische Ansicht eines Lüfterrades.

In den unterschiedlichen Figuren sind hinsichtlich ihrer Funktion gleichwertige Teile stets mit denselben Bezugszeichen versehen, so dass diese in der Regel auch nur einmal beschrieben werden.

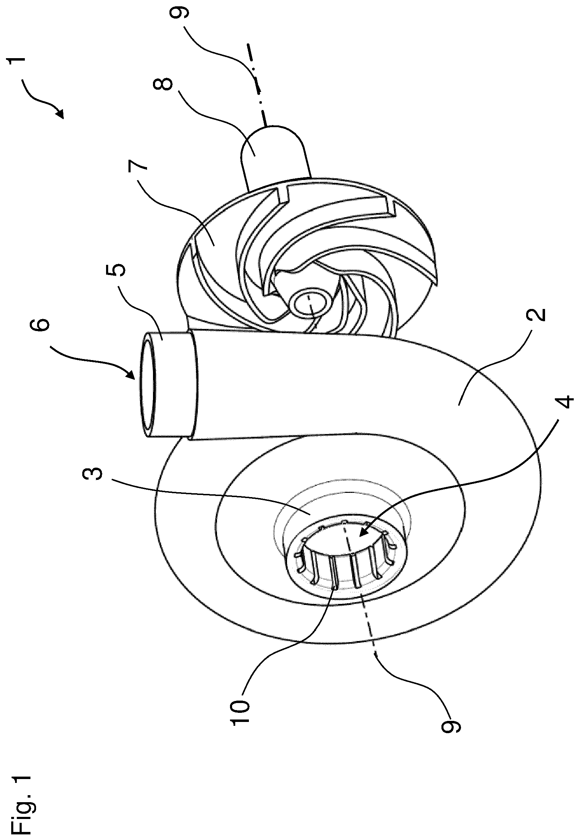

Fig. 1 stellt eine Explosionsansicht in perspektivischer Darstellung eines Ausführungsbeispiels einer Lüftereinheit 1 gemäß der Erfindung dar. Die Lüftereinheit 1 kann besonders bevorzugt in einem Beatmungsgerät (nicht weiter dargestellt) zur Beatmung von Personen verwendet werden. Wie in Fig. 1 zu erkennen ist, weist die gezeigte Lüftereinheit 1 ein Gehäuse 2 mit einem Saugstutzen 3 auf, der einen Einlasskanal 4 zum saugseitigen Einströmen eines Fluids, insbesondere eines Atemgases, in das Gehäuse 2 bildet. Weiterhin weist die Lüftereinheit 1 bzw. das Gehäuse 2 einen Druckstutzen 5 auf, der einen Auslasskanal 6 zum druckseitigen Ausströmen des Fluids aus dem Gehäuse 2 bildet. Ferner ist Fig. 1 zu entnehmen, dass die Lüftereinheit 1 ein in dem Gehäuse 2 drehbar gelagertes Lüfterrad 7 aufweist, welches durch Rotation das Fluid über den Saugstutzen 3 ansaugt und durch den Einlasskanal 4 in das Gehäuse 2 fördert und anschließend das Fluid über den Druckstutzen 5 wieder ausstößt und durch den Auslasskanal 6 aus dem Gehäuse fördert. Hierzu ist das Lüfterrad 7 im Wesentlichen vollständig in dem Gehäuse 2 aufgenommen. Die Rotation wird bei dem in Fig. 1 gezeigten Ausführungsbeispiel der Lüftereinheit 1 über einen Antriebsmotor 8, insbesondere einen Elektromotor 8 bewirkt, was jedoch nicht zwingend erforderlich ist. Zudem kann der Elektromotor 8 ebenfalls im Wesentlichen vollständig in dem Gehäuse 2 aufgenommen sein oder lediglich teilweise oder auch vollständig außerhalb des Gehäuses 2 angeordnet und in antreibender Weise mit dem Lüfterrad 7 verbunden sein. Wie Fig. 1 außerdem zu entnehmen ist, ist die Lüftereinheit 1 eine Radiallüftereinheit, das heißt das Lüfterrad 7 ist derart im Gehäuse 2 angeordnet, dass das Fluid über den Saugstutzen 3 in Axialrichtung des Lüfterrads 7 angesaugt wird und in Radialrichtung ausgestoßen wird. Die Axialrichtung des Lüfterrads 7 fällt bei der in Fig. 1 dargestellten Lüftereinheit 1 mit der Mittelachse 9 der Lüftereinheit 1 bzw. des Saugstutzens 3 zusammen, ohne jedoch hierauf beschränkt zu sein.

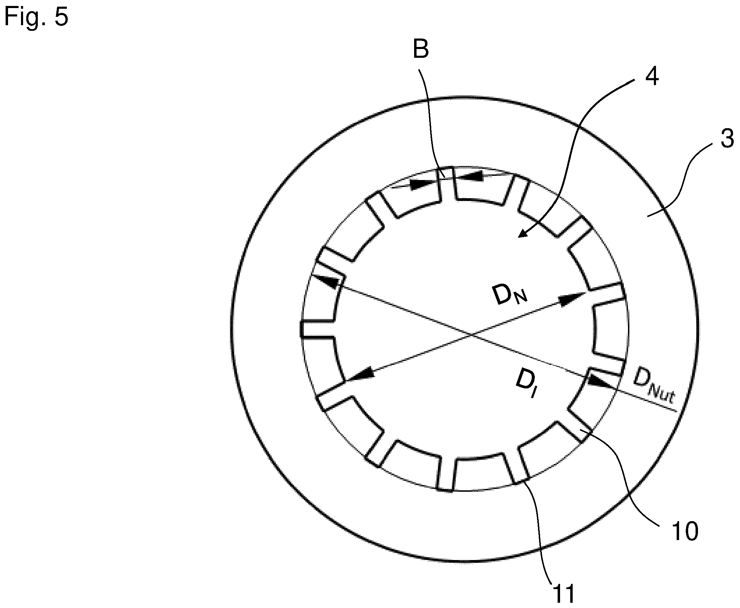

Weiterhin ist Fig. 1 zu entnehmen, dass der Saugstutzen 3 an seiner dem Einlasskanal 4 zugewandten Innenfläche, die einen Einlasskanalnenndurchmesser D_{N} (s. Fig. 5) festlegt bzw. bestimmt, wenigstens ein, vorliegend mehrere, Strömungsleitelemente 10 aufweist. Bei der in Fig. 1 gezeigten Lüftereinheit 1 sind die Strömungsleitelemente 10 als Nuten ausgebildet. Die Strömungsleitelemente 10 bzw. Nuten 10 legen einen Einlasskanalinnendurchmesser D_{I} fest, der vom Einlasskanalnenndurchmesser D_{N} abweicht, das heißt vorliegend größer ist als der Einlasskanalnenndurchmesser D_{N}. Die mehreren Nuten 10 sind äquidistant über den Umfang der Innenfläche des Saugstutzens 3 verteilt angeordnet, wie in Fig. 1 deutlich zu sehen ist.

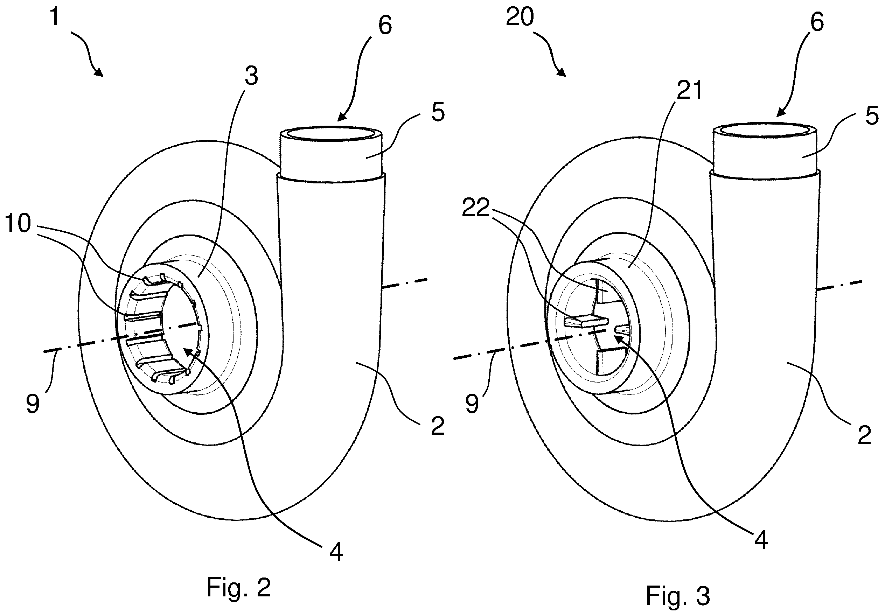

Fig. 2 stellt eine perspektivische Teilansicht der Lüftereinheit 1 mit dem Gehäuse 2 aus Fig. 1 dar.

Fig. 3 stellt zum Vergleich mit der Lüftereinheit 1 aus Fig. 2 eine perspektivische Teilansicht eines weiteren Ausführungsbeispiels einer Lüftereinheit 20 gemäß der Erfindung dar. Im Unterschied zur Lüftereinheit 1 aus Fig. 2 sind bei einem Saugstutzen 21 der Lüftereinheit 20 mehrere Strömungsleitelemente 22 als von der Innenfläche des Saugstutzens 21 hervorstehende Rippen 22 ausgebildet. Insgesamt sind bei dem in Fig. 3 gezeigten Ausführungsbeispiel der Lüftereinheit 20 vier solcher Rippen 22 dargestellt, ohne auf diese Anzahl beschränkt zu sein. Die Strömungsleitelemente 22 bzw. Rippen 22 legen einen Einlasskanalinnendurchmesser D_{I} fest, der vom Einlasskanalnenndurchmesser D_{N} abweicht, das heißt, der bei der vorliegenden Lüftereinheit 20 kleiner ist als der Einlasskanalnenndurchmesser D_{N}. Die (vorliegend vier) Rippen 22 sind äquidistant über den Umfang der Innenfläche des Saugstutzens 21 verteilt angeordnet, wie in Fig. 3 deutlich zu erkennen ist. Da sich die Rippen 22 der Lüftereinheit 20 in radialer Richtung nicht bis zur Mittelachse 9 des Saugstutzens 21 erstrecken und dementsprechend dort nicht miteinander in Kontakt stehen, ist der durch die Rippen 22 der Lüftereinheit 20 festgelegte Einlasskanalinnendurchmesser D_{I} größer Null.

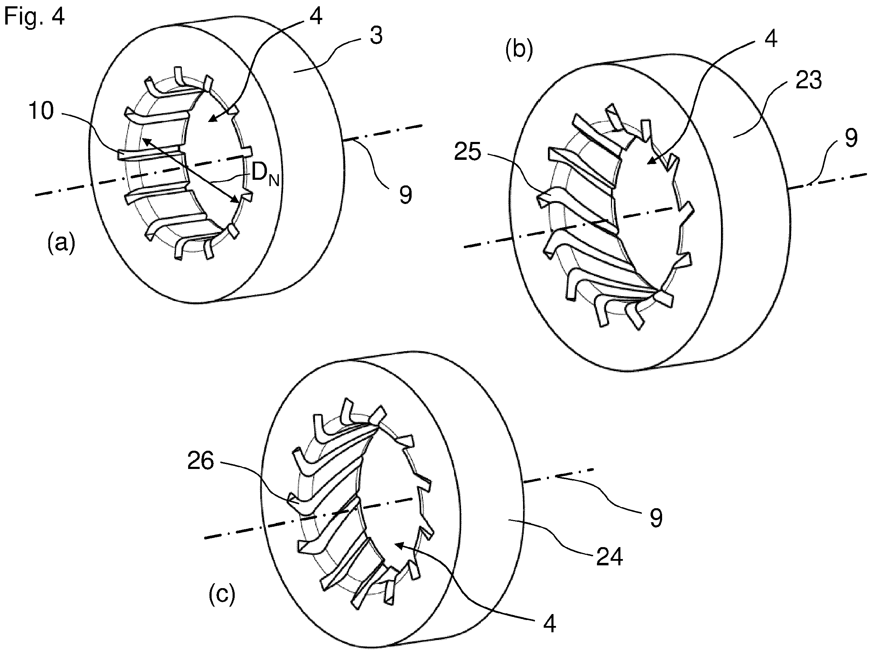

Fig. 4 stellt eine perspektivische Detailansicht (a) des Saugstutzens 3 der Lüftereinheit 1 aus Fig. 2 dar, ferner eine perspektivische Detailansicht (b) eines Saugstutzens 23 eines weiteren Ausführungsbeispiels einer Lüftereinheit gemäß der Erfindung (nicht weiter dargestellt) sowie eine perspektivische Detailansicht (c) eines Saugstutzens 24 eines noch weiteren Ausführungsbeispiels einer ebenfalls nicht näher dargestellten Lüftereinheit gemäß der Erfindung. Die die Saugstutzen 23 und 24 jeweils aufweisenden, vorliegend nicht dargestellten Lüftereinheiten können bis auf den jeweiligen Saugstutzen 23 bzw. 24 identisch zur Lüftereinheit 1 aus Fig. 1 aufgebaut sein.

Bei dem Saugstutzen 3 der Lüftereinheit 1 ist in Fig. 4(a) deutlich zu erkennen, dass sich die als Nuten ausgebildeten Strömungsleitelemente 10 in ihren jeweiligen Längserstreckungsrichtungen parallel zur Mittelachse 9 des Saugstutzens 3 erstrecken, dementsprechend also achsparallel verlaufen. Diese Ausgestaltung hat sich als besonders effizient hinsichtlich einer möglichst großen Druckerhöhung für das im Einlasskanal 4 strömende Fluid erwiesen. Die Strömungsleitelemente können in ihren jeweiligen Längserstreckungsrichtungen einen geraden Verlauf haben.

Der Saugstutzen 23 aus Fig. 4(b) weist zwar ebenfalls als Nuten ausgebildete Strömungsleitelemente 25 auf, diese verlaufen jedoch in ihren Längserstreckungsrichtungen nicht achsparallel zur Mittelachse 9, sondern sind in einem Winkel gegenüber der Mittelachse 9 des Saugstutzens 23 geneigt. Der Neigungswinkel der Nuten 25 gegenüber dem Verlauf der Mittelachse 9 beträgt vorzugsweise mehr als 0 ° (0 ° entspricht der achsparallelen Ausrichtung) und kleiner gleich 45 °. Die Strömungsleitelemente können in ihren jeweiligen Längserstreckungsrichtungen einen gebogenen oder geknickten Verlauf haben. Das strömende Fluid kann durch den gebogenen oder geknickten Verlauf der Strömungsleitelemente einen Impuls oder Drall erfahren.

Beim Saugstutzen 24 in Fig. 4(c) sind die wiederum als Nuten ausgebildeten Strömungsleitelemente 26 ebenfalls in einem Winkel geneigt zur Mittelachse 9 ausgerichtet, im Vergleich zu den Nuten 25 des Saugstutzens 23 aus Fig. 4(b) jedoch genau in die entgegengesetzte Richtung geneigt. Dementsprechend kann bei dieser Ausgestaltung ein bevorzugter Neigungswinkel als ein Winkel zwischen kleiner 0 ° und größer gleich -45 °C definiert werden.

Fig. 5 zeigt eine Querschnittansicht durch den Saugstutzen 3 der Lüftereinheit 1 aus Fig. 2. Deutlich ist in Fig. 5 der durch die Innenfläche des Saugstutzens 3 definierte Einlasskanalnenndurchmesser D_{N} zu erkennen. Die in die Innenfläche eingebrachten Strömungsleitelemente 10 in Form von Nuten legen einen Einlasskanalinnendurchmesser D_{I} fest, der bei dem Saugstutzen 10 der Lüftereinheit 1 aus Fig. 2 größer ist als der Einlasskanalnenndurchmesser D_{N} und durch den Nutgrund 11 der jeweiligen Nut 10 begrenzt ist. Des Weiteren ist in der Querschnittsansicht der Fig. 5 eine Breite B eines der Strömungsleitelemente 10, das heißt seine Ausdehnung in tangentialer Richtung des vorliegend hohlzylinderförmig ausgebildeten Saugstutzens 3 bzw. seiner Innenfläche, angegeben. Die Breite B des Strömungsleitelements 10 stellt bei dem gezeigten Ausführungsbeispiel eine Nutbreite B der Nuten 10 dar. Es ist zu verstehen, dass die Breite des Strömungsleitelements 22 im Fall der in Fig. 3 dargestellten Lüftereinheit 20 auch als Steg- oder Rippenbreite verstanden werden kann. Die Strömungselementbreite stellt dementsprechend allgemein die Erstreckungsrichtung der jeweiligen Strömungsleitelemente 10, 22, 25, 26 in tangentialer Richtung der jeweiligen Saugstutzen 3, 21, 23 bzw. 24 dar.

Es hat sich herausgestellt, dass bei Ausgestaltung der Strömungsleitelemente als Nuten wie im Fall der achsparallel ausgerichteten Nuten 10 der Lüftereinheit 1 ein maximaler Druckgewinn bzw. Druckanstieg im Einlasskanal 4 bei gleichbleibender Drehzahl des Lüfterrads 7 gegenüber herkömmlichen Saugstutzen ohne Strömungsleitelemente erzielbar ist, wenn insgesamt 13 äquidistant entlang des Innenumfangs der Innenfläche des Saugstutzens 3 verteilt angeordnete, achsparallele Nuten 10 mit jeweils einer Breite B von 0,3 bis 2,1 mm, bevorzugt 0,5 bis 1,5 mm beispielsweise auch 0,8 bis 1,2 mm vorgesehen sind. Erfindungsgemäß ist die Breite B immer auch im Verhältnis zur Anzahl der Nuten oder Rippen angepasst. Erfindungsgemäß ist die Relation der Gesamtbreite B der Rippen (beispielsweise 8 Rippen a 1mm) zum freien Durchmesser des Einströmkanals 8mm zu 16mm, bzw. das Verhältnis aus Einströmkanal-Fläche zu versperrter Fläche durch die Rippen ist bevorzugt im Bereich 1,1 bis 1,6, beispielsweise auch 1,2 - 1,4 oder ca. 1,2.

Das Durchmesserverhältnis des Einlasskanalinnendurchmessers D_{I} zum Einlasskanalnenndurchmesser D_{N} beträgt 1,05 bis 1,6, bevorzugt 1,1 bis 1,4, besonders bevorzugt 1,2 bis 1,3.

Bei als Rippen ausgestalteten Strömungsleitelementen, wie beispielsweise bei der in Fig. 3 gezeigten Lüftereinheit 20, kann ein maximaler Druckgewinn im Einlasskanal 4 mit insgesamt 8 äquidistant in Umfangsrichtung entlang der Innenfläche des Saugstutzens 21 verteilt angeordneten Rippen 22 erzielt werden.

Generell könnten die Rippen 22 jede erdenkliche Form annehmen.

Die Stirnseite 22a der Rippen ist beispielsweise auf der Seite, die dem einströmenden Fluid zugewandt ist, abgerundet.

Die Rippen können eckig und/oder abgerundet am Ein- und Austritt des Fluids sein.

Die Rippen können sich vom Eintritt zum Austritt des Fluids hin verjüngen (oder vice versa) und somit einen Verlauf der Breite B aufweisen.

Die Rippen können symmetrisch tragflügelprofiliert sein.

Die Rippen können als gewölbter Tragflügel ausgeführt sein.

Die Rippen können mit Einkerbungen über die axiale Lauflänge ausgeführt sein.

Die Rippen können mit Durchbrüchen/ Löchern in den Rippen ausgeführt sein, sodass eine Austauschströmung von einem Rippenströmungskanal zum nächsten entsteht.

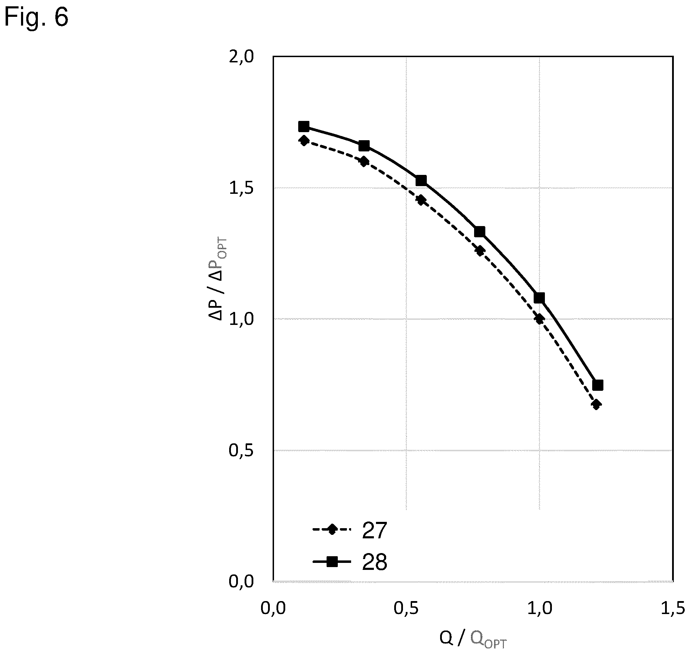

Fig. 6 stellt ein Druckdiagramm dar, das einen Vergleich der Lüftereinheit 1 aus Fig. 2 mit einer Lüftereinheit nach dem Stand der Technik zeigt, wobei die Lüftereinheit 1 wie vorstehend beschrieben und in Fig. 5 gezeigt 13 gleichmäßig an der Innenfläche des Saugstutzens 3 verteilt angeordnete, achsparallele Nuten 10 als Strömungsleitelemente aufweist.

Entlang der Ordinate des Druckdiagramms ist der auf einen optimalen Betriebspunkt der hierin nicht dargestellten Lüftereinheit nach dem Stand der Technik, d. h. eine Lüftereinheit mit einem Saugstutzen ohne Strömungsleitelemente im Sinne der Erfindung, normierte Druck Δp/Δpₒₚₜ aufgetragen, entlang der Abszisse der auf einen optimalen Betriebspunkt der Lüftereinheit nach dem Stand der Technik normierte Volumenstrom Q/Qₒₚₜ. Eine Druckkurve der herkömmlichen Lüftereinheit gemäß Stand der Technik ist in dem Druckdiagramm als gestrichelte Kurve 27 eingezeichnet, eine Druckkurve der Lüftereinheit 1 gemäß einem hierin offenbarten Ausführungsbeispiel der Erfindung als durchgezogene Kurve 28.

In Fig. 6 ist deutlich zu erkennen, dass bei Q/Qₒₚₜ = 1,0 der optimale Druck Δp/Δpₒₚₜ (ebenfalls gleich 1,0) der herkömmlichen Lüftereinheit (Kurve 27) erreicht wird. Hingegen erreicht die Kurve 28 der Lüftereinheit 1 gemäß dem hierin beschriebenen Ausführungsbeispiel der Erfindung bei demselben Volumenstrom (= selbe Drehzahl des Lüfterrads 7) einen deutlich höheren Druck, dementsprechend Δp/Δpₒₚₜ > 1,0, vorliegend etwa Δp/Δpₒₚₜ = 1,1. Somit lässt sich alleine durch Vorsehen der Strömungsleitelemente 10 im Einlasskanal 4 eine Druckerhöhung des Fluids bei derselben Drehzahl des Lüfterrads 7 erreichen.

Die hierin offenbarte erfindungsgemäße Lüftereinheit ist nicht auf die hierin offenbarten Ausführungsformen beschränkt, sondern umfasst auch gleichwirkende weitere Ausführungsformen, die sich aus technisch sinnvollen weiteren Kombinationen der hierin beschriebenen Merkmale der Lüftereinheit ergeben. Insbesondere sind die hierin vorstehend in der allgemeinen Beschreibung und der Figurenbeschreibung genannten und/oder in den Figuren alleine gezeigten Merkmale und Merkmalskombinationen nicht nur in den jeweils hierin explizit angegebenen Kombinationen, sondern auch in anderen Kombinationen oder in Alleinstellung verwendbar, ohne den Rahmen der vorliegenden Erfindung zu verlassen. - Die Erfindung ist durch die beigefügten Ansprüche definiert.

In bevorzugter Ausführung wird die erfindungsgemäße Lüftereinheit zum Betreiben von Beatmungsgeräten zur Beatmung von Personen, beispielsweise Personen mit unzureichender oder ausgesetzter Eigenatmung, verwendet, indem ein Fluid, insbesondere ein Atemgas, mittels der Lüftereinheit gefördert, das heißt saugseitig angesaugt und druckseitig mit einem vorherbestimmten Druck wieder abgegeben wird, und anschließend der das Beatmungsgerät verwendenden Person zugeführt wird.

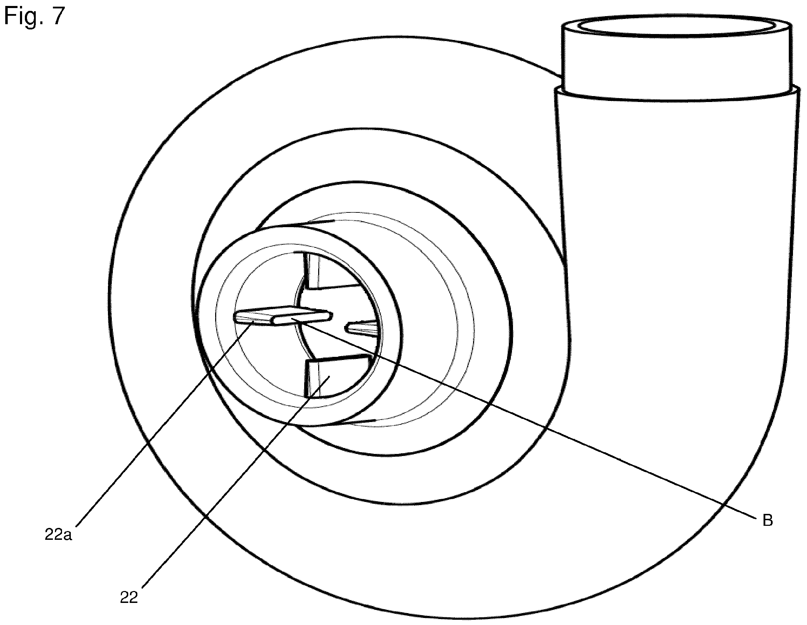

Fig. 7 zeigt bei als Rippen ausgestalteten Strömungsleitelementen, wie beispielsweise entsprechend der in Fig. 3 gezeigten Lüftereinheit 20, kann ein maximaler Druckgewinn im Einlasskanal 4 mit insgesamt 2 - 30 äquidistant in Umfangsrichtung entlang der Innenfläche des Saugstutzens 21 verteilt angeordneten Rippen 22 erzielt werden.

Generell könnten die Rippen 22 jede erdenkliche Form annehmen.

Die Stirnseite 22a der Rippen ist beispielsweise auf der Seite, die dem einströmenden Fluid zugewandt ist, abgerundet.

Die Rippen können eckig und/oder abgerundet am Ein- und Austritt des Fluids sein.

Die Rippen können sich vom Eintritt zum Austritt des Fluids hin verjüngen (oder vice versa) und somit einen Verlauf der Breite B aufweisen.

Die Rippen können symmetrisch tragflügelprofiliert sein.

Die Rippen können als gewölbter Tragflügel ausgeführt sein.

Die Rippen können mit Einkerbungen über die axiale Lauflänge ausgeführt sein.

Die Rippen können mit Durchbrüchen/ Löchern in den Rippen ausgeführt sein, sodass eine Austauschströmung von einem Rippenströmungskanal zum nächsten entsteht.

Die Ausführungen der Fig. 7 sind ebenfalls auf Nuten übertragbar.

Generell könnten die Strömungsleitelemente jede erdenkliche Form annehmen.

Die Stirnseite 22a der Strömungsleitelemente ist beispielsweise auf der Seite, die dem einströmenden Fluid zugewandt ist, abgerundet.

Die Strömungsleitelemente können eckig und/oder abgerundet am Ein- und Austritt des Fluids sein.

Die Strömungsleitelemente können sich vom Eintritt zum Austritt des Fluids hin verjüngen (oder vice versa) und somit einen Verlauf der Breite B aufweisen.

Die Strömungsleitelemente können symmetrisch tragflügelprofiliert sein.

Die Strömungsleitelemente können als gewölbter Tragflügel ausgeführt sein.

Die Strömungsleitelemente können mit Einkerbungen über die axiale Lauflänge ausgeführt sein.

Die Strömungsleitelemente können mit Durchbrüchen/ Löchern in den Strömungsleitelementen ausgeführt sein, sodass eine Austauschströmung von einem Strömungskanal zum nächsten entsteht.

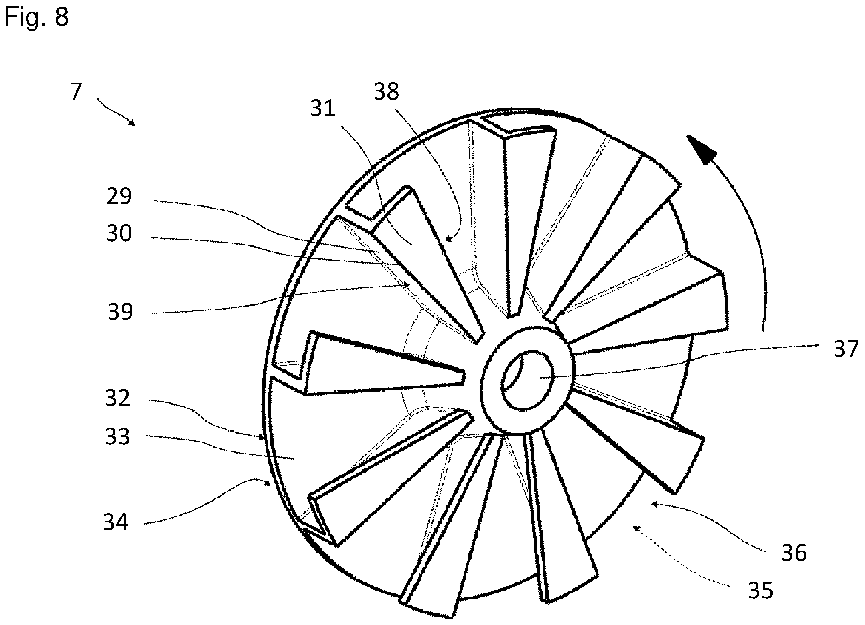

Die Figur 8 zeigt ein Lüfterrad 7 in einer perspektivischen Ansicht. Das Lüfterrad 7 ist hier mit einer Mehrzahl von Schaufelelementen 29 und einer als Tragscheibe 33 ausgebildeten Scheibe 32 sowie einer Nabe 37 zur Verbindung mit einer Antriebswelle ausgestattet. Die Schaufelelemente 29 sind hier an der Tragscheibe 33 und vorzugsweise auch an der Nabe 37 befestigt und beispielsweise einstückig mit diesen verbunden. Das Lüfterrad 7 kann auch ohne Scheiben 32 und insbesondere ohne Tragscheibe 33 ausgeführt sein, beispielsweise als Sternenlüfterrad.

Die bevorzugte Drehrichtung des Lüfterrades 7 ist hier durch einen Pfeil veranschaulicht. Die Schaufelelemente 29 weisen eine Saugseite 38 und eine Druckseite 39 auf. Für die hier gezeigte Drehrichtung des Lüfterrades 7 ergibt sich die hier gezeigte Ausrichtung von Saugseite 38 und Druckseite 39.

Um die Austauschströmung aus dem Seitenraum sowie von der Druckseite 39 zur Saugseite 38 in Richtung des Seitenraumes bzw. einer axialen Seite wirksam und zuverlässig zu unterbinden, ist das Lüfterrad 7 mit Winglets 31 ausgestattet. Dabei ist hier jedes Schaufelelement 29 mit einem Winglet 31 ausgestattet. Die Winglets 31 verlaufen jeweils entlang einer axialen Längsseite 30 des Schaufelelements 29. In der hier gezeigten Ausgestaltung der Erfindung zeigen die Winglets 31 in Richtung der Saugseite 38 des jeweiligen Schaufelelements 29. Für ein solches Lüfterrad 7 konnte sowohl eine vorteilhafte Drucksteigerung als auch eine erhebliche Wirkungsgradsteigerung beobachtet werden.

Das hier gezeigte Lüfterrad 7 ist beispielsweise so in die Lüftereinheit 1 integriert, dass von axial angesaugt und nach radial ausgeblasen wird. Dabei liegt die Ansaugseite an der axialen Seite 36 mit den Winglets 31.

In der hier gezeigten Ausgestaltung ist das Lüfterrad 7 nur an einer axialen Seite 34 mit der Scheibe 32 ausgestattet. Dabei ist die als Tragscheibe 33 ausgebildete Scheibe 32 an derjenigen axialen Seite 34 des Lüfterrades 7 angeordnet, welche der mit den Winglets 31 ausgestatteten axialen Seite 36 des Lüfterrades 7 gegenüberliegt.

In einer Weiterbildung kann auch eine hier nicht näher dargestellte und als Deckscheibe 35 ausgebildete Scheibe 32 vorgesehen sein. Dabei kann die Deckscheibe 35 zusätzlich zu der Tragscheibe 33 an der axialen Seite 36 angeordnet sein oder die Tragscheibe 33 ersetzen.

### Bezugszeichenliste

- 1: Lüftereinheit
- 2: Gehäuse
- 3: Saugstutzen
- 4: Einlasskanal
- 5: Druckstutzen
- 6: Auslasskanal
- 7: Lüfterrad
- 8: Antriebsmotor
- 9: Mittelachse
- 10: Strömungsleitelement / Nut
- 20: Lüftereinheit
- 21: Saugstutzen
- 22: Strömungsleitelement / Rippe
- 23: Saugstutzen
- 24: Saugstutzen
- 25: Strömungsleitelement / Nut
- 26: Strömungsleitelement / Nut
- 27: Druckkurve einer Lüftereinheit nach Stand der Technik
- 28: Druckkurve von 1
- 29: Schaufelelement
- 30: Längsseite
- 31: Winglet
- 32: Scheibe
- 33: Tragscheibe
- 34: Seite
- 35: Deckscheibe
- 36: Seite
- 37: Nabe
- 38: Saugseite
- 39: Druckseite
- B: Breite eines Strömungsleitelements
- D_{I}: Einlasskanalinnendurchmesser
- D_{N}: Einlasskanalnenndurchmesser
- Δp: Druck
- Δpₒₚₜ: Druck bei optimalem Betriebspunkt einer Lüftereinheit nach Stand der Technik
- Q: Volumenstrom
- Qₒₚₜ: Volumenstrom bei optimalem Betriebspunkt einer Lüftereinheit nach Stand der Technik

## Patentansprüche

1. Lüftereinheit für ein Beatmungsgerät zum Betreiben von dem Beatmungsgerät zur Beatmung von Personen, aufweisend ein Gehäuse (2) mit einem Saugstutzen (3, 21, 23, 24), der einen Einlasskanal (4) zum saugseitigen Einströmen eines Fluids in das Gehäuse (2) bildet, und einem Druckstutzen (5), der einen Auslasskanal (6) zum druckseitigen Ausströmen des Fluids aus dem Gehäuse (2) bildet, sowie ein in dem Gehäuse (2) drehbar gelagertes Lüfterrad (7), das ausgebildet und angeordnet ist, durch Rotation das Fluid über den Saugstutzen (3, 21, 23, 24) anzusaugen und durch den Einlasskanal (4) in das Gehäuse (2) zu fördern sowie das Fluid über den Druckstutzen (5) wieder auszustoßen und durch den Auslasskanal (6) aus dem Gehäuse (2) zu fördern, **dadurch gekennzeichnet, dass** der Saugstutzen (3, 21, 23, 24) an seiner dem Einlasskanal (4) zugewandten, einen Einlasskanalnenndurchmesser (D_{N}) bestimmenden Innenfläche wenigstens ein Strömungsleitelement (10, 22, 25, 26) aufweist, mittels welchem ein vom Einlasskanalnenndurchmesser (D_{N}) zumindest abschnittsweise abweichender Einlasskanalinnendurchmesser (D_{I}) festgelegt ist, so dass eine Druckerhöhung des durch das Lüfterrad (7) angesaugten Fluids erzielt wird.

2. Lüftereinheit nach Anspruch 1, **dadurch gekennzeichnet, dass** das Durchmesserverhältnis des Einlasskanalinnendurchmessers D_{I} zum Einlasskanalnenndurchmessers D_{N} 1,05 bis 1,6, bevorzugt 1,1 bis 1,4, besonders bevorzugt 1,2 bis 1,3 beträgt.

3. Lüftereinheit nach Anspruch 1, **dadurch gekennzeichnet, dass** das Strömungsleitelement (10, 25, 26) eine in die Innenfläche eingebrachte Nut ist, die den Einlasskanalinnendurchmesser (D_{I}) gegenüber dem Einlasskanalnenndurchmesser (D_{N}) vergrößert.

4. Lüftereinheit nach Anspruch 1 oder 2, **dadurch gekennzeichnet, dass** das Strömungsleitelement (22) eine von der Innenfläche hervorstehende Rippe ist, die den Einlasskanalinnendurchmesser (D_{I}) gegenüber dem Einlasskanalnenndurchmesser (D_{N}) verkleinert.

5. Lüftereinheit nach einem der vorhergehenden Ansprüche, **dadurch gekennzeichnet, dass** mehrere Strömungsleitelemente (10, 22, 25, 26) äquidistant über den Umfang der Innenfläche verteilt angeordnet sind.

6. Lüftereinheit nach einem der vorhergehenden Ansprüche, **dadurch gekennzeichnet, dass** zumindest ein Strömungsleitelement eine Stirnseite 22a aufweist und die Stirnseite 22a auf der Seite, die dem einströmenden Fluid zugewandt ist, abgerundet ist.

7. Lüftereinheit nach einem der vorhergehenden Ansprüche, **dadurch gekennzeichnet, dass** zumindest ein Strömungsleitelement zwei Stirnseiten aufweist und die Stirnseite 22a auf der Seite, die dem einströmenden Fluid zugewandt ist, abgerundet oder eckig ist und die Stirnseite auf der Seite, die dem einströmenden Fluid abgewandt ist, abgerundet oder eckig ist.

8. Lüftereinheit nach einem der vorhergehenden Ansprüche, **dadurch gekennzeichnet, dass** zumindest ein Strömungsleitelement symmetrisch tragflügelprofiliert ist.

9. Lüftereinheit nach einem der vorhergehenden Ansprüche, **dadurch gekennzeichnet, dass** zumindest ein Strömungsleitelement als gewölbter Tragflügel ausgeführt ist.

10. Lüftereinheit nach einem der vorhergehenden Ansprüche, **dadurch gekennzeichnet, dass** zumindest ein Strömungsleitelement mit Einkerbungen über die axiale Länge ausgeführt ist.

11. Lüftereinheit nach einem der vorhergehenden Ansprüche, **dadurch gekennzeichnet, dass** zumindest ein Strömungsleitelement mit Durchbrüchen/ Löchern in dem Strömungsleitelement ausgeführt ist, sodass eine Austauschströmung von einem Strömungskanal zum nächsten entsteht.

12. Lüftereinheit nach einem der vorhergehenden Ansprüche, **dadurch gekennzeichnet, dass** zumindest ein Strömungsleitelement sich in der Strömungsrichtung des Fluids verjüngen (oder vice versa).

13. Lüftereinheit nach einem der vorhergehenden Ansprüche, **dadurch gekennzeichnet, dass** zumindest ein Strömungsleitelement eine Breite B aufweist, das heißt seine Ausdehnung in tangentialer Richtung des Saugstutzens 3 bzw. seiner Innenfläche.

14. Lüftereinheit nach einem der vorhergehenden Ansprüche, **dadurch gekennzeichnet, dass** zumindest ein Strömungsleitelement eine Breite B mit einem Verlauf der Breite B aufweist.

15. Lüftereinheit nach einem der vorhergehenden Ansprüche, **dadurch gekennzeichnet, dass** zumindest ein Strömungsleitelement eine Breite B aufweist, die im Bereich 0,4 mm bis 2,2 mm liegt.

16. Lüftereinheit nach beiden vorhergehenden Ansprüchen, **dadurch gekennzeichnet, dass** sich die Rippen (22) in radialer Richtung bis zur Mittelachse (9) des Saugstutzens (21) erstrecken und dort miteinander in Kontakt stehen.

17. Lüftereinheit nach einem der vorhergehenden Ansprüche, **dadurch gekennzeichnet, dass** sich das Strömungsleitelement (10, 22, 25, 26) in seiner Längserstreckungsrichtung parallel zur Mittelachse (9) des Saugstutzens (3, 21, 23, 24) erstreckt.

18. Lüftereinheit nach einem der Ansprüche 1 bis 5, **dadurch gekennzeichnet, dass** sich das Strömungsleitelement (10, 22, 25, 26) in seiner Längserstreckungsrichtung in einem Winkel geneigt zur Mittelachse (9) des Saugstutzens (3, 21, 23, 24) erstreckt.

19. Lüftereinheit nach dem vorhergehenden Anspruch, **dadurch gekennzeichnet, dass** der Winkel größer 0 ° und kleiner gleich 45 ° ist.

20. Lüftereinheit nach einem der vorhergehenden Ansprüche, **dadurch gekennzeichnet, dass** das Verhältnis zwischen dem Einlasskanalinnendurchmesser (D_{I}) und dem Einlasskanalnenndurchmesser (D_{N}) zwischen 0,6 und 1,4, bevorzugt zwischen 0,7 und 1,3, noch bevorzugter zwischen 0,8 und 1,25, liegt.

21. Lüftereinheit nach einem der vorhergehenden Ansprüche, **dadurch gekennzeichnet, dass** die Lüftereinheit (1, 20) eine Radiallüftereinheit ist.

22. Lüftereinheit nach zumindest einem der vorhergenenden Ansprüche, **dadurch gekennzeichnet, dass** das Lüfterrad (7) eine Mehrzahl an Schaufelelementen (29) aufweist, wobei die Schaufelelemente (29) wenigstens teilweise mit jeweils einem an wenigstens einer axialen Längsseite (30) des Schaufelelements (29) wenigstens abschnittsweise verlaufenden Winglet (31) ausgestattet sind.

23. Lüftereinheit nach zumindest einem der vorhergehenden Ansprüche, **dadurch gekennzeichnet, dass** das Winglet (31) eine Ausdehnung von 1° bis 20° des Umfangs des Lufterrades (7) aufweist.

24. Lüftereinheit nach zumindest einem der vorhergehenden Ansprüche, **dadurch gekennzeichnet, dass** die Ausdehnung des Winglets (31) von radial innen des Lüfterrades (7) nach radial außen des Lüfterrades (7) zunimmt.

25. Lüftereinheit nach zumindest einem der vorhergehenden Ansprüche, **dadurch gekennzeichnet, dass** das Lüfterrad (7) an nur einer axialen Seite mit wenigstens einer Scheibe (32) ausgestattet ist und die Scheibe (32) nur an derjenigen axialen Seite angeordnet ist, welche einer mit den Winglets (31) ausgestatteten axialen Seite des Lüfterrades (7) gegenüberliegt.

## Claims

1. A ventilation unit for a ventilator for operating the ventilator to ventilate patients, having a housing (2) with a suction port (3, 21, 23, 24) that forms an inlet channel (4) for suction-side inflow of a fluid into the housing (2), and a pressure port (5) that forms an outlet channel (6) for pressure-side outflow of the fluid from the housing (2), and a fan wheel (7) which is rotatably mounted in the housing (2) and which is designed and arranged to take in the fluid via the suction port (3, 21, 23, 24) due to rotation and convey it through the inlet channel (4) into the housing (2) and to expel the fluid again via the pressure port (5) and to convey it out of the housing (2) through the outlet channel (6), **characterized in that** the suction port (3, 21, 23, 24), on its inner surface that faces the inlet channel (4) and determines an inlet channel nominal diameter (D_{N}), has at least one flow-guiding element (10, 22, 25, 26), by means of which an inlet channel inner diameter (D₁) deviating at least in portions from the inlet channel nominal diameter (D_{N}) is established such that a pressure increase of the fluid taken in by the fan wheel (7) is achieved.

2. The ventilation unit according to claim 1, **characterized in that** the diameter ratio of the inlet channel inner diameter (D₁) to the inlet channel nominal diameter (D_{N}) is 1.05 to 1.6, preferably 1.1 to 1.4, particularly preferably 1.2 to 1.3.

3. The ventilation unit according to claim 1, **characterized in that** the flow-guiding element (10, 25, 26) is a groove which is introduced into the inner surface and which enlarges the inlet channel inner diameter (D₁) with respect to the inlet channel nominal diameter (D_{N}).

4. The ventilation unit according to claim 1 or 2, **characterized in that** the flow-guiding element (22) is a rib which protrudes from the inner surface and which decreases the inlet channel inner diameter (D₁) with respect to the inlet channel nominal diameter (D_{N}).

5. The ventilation unit according to one of the preceding claims, **characterized in that** multiple flow-guiding elements (10, 22, 25, 26) are arranged so as to be distributed equidistantly over the circumference of the inner surface.

6. The ventilation unit according to one of the preceding claims, **characterized in that** at least one flow-guiding element has an end face (22a) and the end face (22a) on the side facing the inflowing fluid is rounded.

7. The ventilation unit according to one of the preceding claims, **characterized in that** at least one flow-guiding element has two end faces and the end face (22a) on the side facing the inflowing fluid is rounded or angular and the end face on the side facing away from the inflowing fluid is rounded or angular.

8. The ventilation unit according to one of the preceding claims, **characterized in that** at least one flow-guiding element is symmetrically airfoil-profiled.

9. The ventilation unit according to one of the preceding claims, **characterized in that** at least one flow-guiding element is designed as a bulging airfoil.

10. The ventilation unit according to one of the preceding claims, **characterized in that** at least one flow-guiding element is designed with indentations over the axial length.

11. The ventilation unit according to one of the preceding claims, **characterized in that** at least one flow-guiding element is designed with openings/holes in the flow-guiding element, such that an exchange flow from one flow channel to the next occurs.

12. The ventilation unit according to one of the preceding claims, **characterized in that** at least one flow-guiding element tapers in the flow direction of the fluid (or vice versa).

13. The ventilation unit according to one of the preceding claims, **characterized in that** at least one flow-guiding element has a width B, meaning its extent in the tangential direction of the suction port (3) or, respectively, of its inner surface.

14. The ventilation unit according to one of the preceding claims, **characterized in that** at least one flow-guiding element has a width B with a progression of the width B.

15. The ventilation unit according to one of the preceding claims, **characterized in that** at least one flow-guiding element has a width B which is in the range of 0.4 mm to 2.2 mm.

16. The ventilation unit according to the two preceding claims, **characterized in that** the ribs (22) extend in the radial direction up to the central axis (9) of the suction port (21) and are in contact with each other there.

17. The ventilation unit according to one of the preceding claims, **characterized in that** the flow-guiding element (10, 22, 25, 26) extends, in its direction of longitudinal extent, parallel to the central axis (9) of the suction port (3, 21, 23, 24).

18. The ventilation unit according to one of claims 1 to 5, **characterized in that** the flow-guiding element (10, 22, 25, 26) extends, in its direction of longitudinal extent, at an angle that is tilted relative to the central axis (9) of the suction port (3, 21, 23, 24).

19. The ventilation unit according to the preceding claim, **characterized in that** the angle is greater than 0° and less than or equal to 45°.

20. The ventilation unit according to one of the preceding claims, **characterized in that** the ratio between the inlet channel inner diameter (D₁) and the inlet channel nominal diameter (D_{N}) is between 0.6 and 1.4, preferably between 0.7 and 1.3, even more preferably between 0.8 and 1.25.

21. The ventilation unit according to one of the preceding claims, **characterized in that** the ventilation unit (1, 20) is a radial fan unit.

22. The ventilation unit according to at least one of the preceding claims, **characterized in that** the fan wheel (7) has a plurality of vane elements (29), wherein at least some of the vane elements (29) are equipped in each case with a winglet (31) running at least in portions on at least one axial longitudinal side (30) of the vane element (29).

23. The ventilation unit according to at least one of the preceding claims, **characterized in that** the winglet (31) has an extent from 1° to 20° of the circumference of the fan wheel (7).

24. The ventilation unit according to at least one of the preceding claims, **characterized in that** the extent of the winglet (31) increases from the radially inner region of the fan wheel (7) toward the radially outer region of the fan wheel (7).

25. The ventilation unit according to at least one of the preceding claims, **characterized in that** the fan wheel (7) is equipped with at least one disc (32) on only one axial side and the disc (32) is arranged only on that axial side which is opposite an axial side of the fan wheel (7) equipped with the winglets (31).

## Revendications

1. Unité de ventilation pour un ventilateur destinée à faire fonctionner le ventilateur pour la ventilation de patients, présentant un boîtier (2) avec une tubulure d'aspiration (3, 21, 23, 24) formant un canal d'admission (4) pour l'afflux d'un fluide dans le boîtier (2) côté aspiration, et une tubulure de pression (5) formant un canal de sortie (6) pour l'écoulement du fluide hors du boîtier (2) côté pression, ainsi qu'une roue de ventilation (7) montée de façon rotative dans le boîtier (2), laquelle est conçue et disposée pour aspirer le fluide par rotation par le biais de la tubulure d'aspiration (3, 21, 23, 24) et le transporter vers le boîtier (2) à travers le canal d'admission (4) et pour décharger de nouveau le fluide par le biais de la tubulure de pression (5) et le transporter hors du boîtier (2) à travers le canal de sortie (6), **caractérisée en ce que** la tubulure d'aspiration (3, 21, 23, 24) présente au moins un élément de guidage de flux (10, 22, 25, 26) au niveau de sa surface intérieure tournée vers le canal d'admission (4) déterminant un diamètre nominal de canal d'admission (D_{N}), lequel permet de définir un diamètre intérieur de canal d'admission (D₁) déviant au moins par sections du diamètre nominal de canal d'admission (D_{N}), de manière à obtenir une augmentation de pression du fluide aspiré par la roue de ventilation (7).

2. Unité de ventilation selon la revendication 1, **caractérisée en ce que** le rapport de diamètres du diamètre intérieur de canal d'admission (D_{I}) au diamètre nominal de canal d'admission (D_{N}) s'élève de 1,05 à 1,6, de préférence de 1,1 à 1,4, particulièrement préférentiellement de 1,2 à 1,3.

3. Unité de ventilation selon la revendication 1, **caractérisée en ce que** l'élément de guidage de flux (10, 25, 26) est une rainure intégrée dans la surface intérieure, laquelle agrandit le diamètre intérieur de canal d'admission (D_{I}) par rapport au diamètre nominal de canal d'admission (D_{N}).

4. Unité de ventilation selon la revendication 1 ou 2, **caractérisée en ce que** l'élément de guidage de flux (22) est une nervure faisant saillie à partir de la surface intérieure, laquelle réduit le diamètre intérieur de canal d'admission (D_{I}) par rapport au diamètre nominal de canal d'admission (D_{N}).

5. Unité de ventilation selon l'une des revendications précédentes, **caractérisée en ce que** plusieurs éléments de guidage de flux (10, 22, 25, 26) sont disposés de façon équidistante sur la périphérie de la surface intérieure.

6. Unité de ventilation selon l'une des revendications précédentes, **caractérisée en ce qu'au** moins un élément de guidage de flux présente un côté avant (22a) et le côté avant (22a) est arrondi sur le côté tourné vers le fluide affluant.

7. Unité de ventilation selon l'une des revendications précédentes, **caractérisée en ce qu'au** moins un élément de guidage de flux présente deux côtés avant et le côté avant (22a) est arrondi ou angulaire sur le côté tourné vers le fluide affluant et le côté avant est arrondi ou angulaire sur le côté opposé au fluide affluant.

8. Unité de ventilation selon l'une des revendications précédentes, **caractérisée en ce qu'au** moins un élément de guidage de flux présente une aile portante profilée symétriquement.

9. Unité de ventilation selon l'une des revendications précédentes, **caractérisée en ce qu'au** moins un élément de guidage de flux est réalisé comme une aile portante voûtée.

10. Unité de ventilation selon l'une des revendications précédentes, **caractérisée en ce qu'au** moins un élément de guidage de flux est réalisé avec des encoches sur la longueur axiale.

11. Unité de ventilation selon l'une des revendications précédentes, **caractérisée en ce qu'au** moins un élément de guidage de flux est réalisé avec des percées et/ou des trous dans l'élément de guidage de flux, de manière à produire un échange de flux d'un canal de flux à l'autre.

12. Unité de ventilation selon l'une des revendications précédentes, **caractérisée en ce qu'au** moins un élément de guidage de flux s'affine dans la direction d'écoulement du fluide (ou vice versa).

13. Unité de ventilation selon l'une des revendications précédentes, **caractérisée en ce qu'au** moins un élément de guidage de flux présente une largeur B, c'est-à-dire son extension dans la direction tangentielle de la tubulure d'aspiration (3) ou de sa surface intérieure.

14. Unité de ventilation selon l'une des revendications précédentes, **caractérisée en ce qu'au** moins un élément de guidage de flux présente une largeur B avec une évolution de la largeur B.

15. Unité de ventilation selon l'une des revendications précédentes, **caractérisée en ce qu'au** moins un élément de guidage de flux présente une largeur B comprise dans la plage de 0,4 mm à 2,2 mm.

16. Unité de ventilation selon les deux revendications précédentes, **caractérisée en ce que** les nervures (22) s'étendent dans la direction radiale jusqu'à l'axe médian (9) de la tubulure d'aspiration (21) et y sont en contact les unes avec les autres.

17. Unité de ventilation selon l'une des revendications précédentes, **caractérisée en ce que** l'élément de guidage de flux (10, 22, 25, 26) s'étend parallèlement à l'axe médian (9) de la tubulure d'aspiration (3, 21, 23, 24) dans sa direction d'extension longitudinale.

18. Unité de ventilation selon l'une des revendications 1 à 5, **caractérisée en ce que** l'élément de guidage de flux (10, 22, 25, 26) s'étend sous un angle incliné par rapport à l'axe médian (9) de la tubulure d'aspiration (3, 21, 23, 24) dans sa direction d'extension longitudinale.

19. Unité de ventilation selon la revendication précédente, **caractérisée en ce que** l'angle est supérieur à 0° et inférieur ou égal à 45°.

20. Unité de ventilation selon l'une des revendications précédentes, **caractérisée en ce que** le rapport entre le diamètre intérieur de canal d'admission (D₁) et le diamètre nominal de canal d'admission (D_{N}) est compris entre 0,6 et 1,4, de préférence entre 0,7 et 1,3, encore plus préférentiellement entre 0,8 et 1,25.

21. Unité de ventilation selon l'une des revendications précédentes, **caractérisée en ce que** l'unité de ventilation (1, 20) est une unité de ventilation radiale.

22. Unité de ventilation selon l'une au moins des revendications précédentes, **caractérisée en ce que** la roue de ventilation (7) présente une pluralité d'éléments d'aubage (29), dans laquelle les éléments d'aubage (29) sont respectivement dotés au moins partiellement d'une ailette (31) évoluant au moins par sections sur au moins un côté longitudinal axial (30) de l'élément d'aubage (29).

23. Unité de ventilation selon l'une au moins des revendications précédentes, **caractérisée en ce que** l'ailette (31) présente une extension de 1° à 20° de la périphérie de la roue de ventilation (7).

24. Unité de ventilation selon l'une au moins des revendications précédentes, **caractérisée en ce que** l'extension de l'ailette (31) augmente à partir du côté radialement intérieur de la roue de ventilation (7) vers le côté radialement extérieur de la roue de ventilation (7).

25. Unité de ventilation selon l'une au moins des revendications précédentes, **caractérisée en ce que** la roue de ventilation (7) est dotée d'au moins un disque (32) uniquement sur un côté axial et le disque (32) est disposé uniquement sur le côté axial opposé à un côté axial de la roue de ventilation (7) doté des ailettes (31).
